Fig. 3

## Europäisches Patentamt

## European Patent Office

## Office européen des brevets

(19)

(11) veröffentlichungsnummer: **0 016 399**

**A1**

(12) **EUROPÄISCHE PATENTANMELDUNG**

(21) Anmeldenummer: **80101224.6**

(51) Int. Cl.³: **A 61 B 10/00**

(22) Anmeldetag: **11.03.80**

(30) Priorität: **22.03.79 DE 2911258**

(43) Veröffentlichungstag der Anmeldung:
**01.10.80 Patentblatt 80/20**

(84) Benannte Vertragsstaaten:
**FR GB IT**

(71) Anmelder: **Chmiel, Horst Prof. Dr.-Ing.**
**Paracelsusstrasse 14**
**D-7250 Leonberg-Ramtel(DE)**

(71) Anmelder: **Mauser, Rudolf**
**Haussmannstrasse 146 B**
**D-7000 Stuttgart 1(DE)**

(72) Erfinder: **Chmiel, Horst Prof. Dr.-Ing.**
**Paracelsusstrasse 14**
**D-7250 Leonberg-Ramtel(DE)**

(72) Erfinder: **Mauser, Rudolf**
**Haussmannstrasse 146 B**
**D-7000 Stuttgart 1(DE)**

(74) Vertreter: **Schmidt, Hans-Ekhardt et al,**
**Robert Bosch GmbH Geschäftsbereich Elektronik**
**Patent- und Lizenzabteilung Forckenbeckstrasse 9-13**
**D-1000 Berlin 33(DE)**

(54) Vorrichtung zum noninvasiven Messen der Blutströmungsgeschwindigkeit nach der Ultraschall-Doppler-Effekt-Methode.

(57) Die Erfindung betrifft eine Vorrichtung zur noninvasiven Messung der Blutströmungsgeschwindigkeit nach der Ultraschall-Doppler-Effekt-Methode unter Verwendung von Ultraschall-Sendern/Empfängern für den am Blut reflektierten Ultraschall und unter Verwendung einer die Dopplerfrequenz-Verschiebung zwischen Sende- und Empfangsfrequenz ermittelnden und auswertenden Einrichtung.

Hierzu werden Ultraschallwellen sehr hoher Frequenz gepulst in das zu untersuchende Kapillargebiet eingestrahlt und Erythrozytenbewegungen bis auf 1 mm/s detektiert. Die Meßtiefeneinstellung erfolgt dabei mittels eines elektronischen Tiefenfensters. Durch die automatisch winkelgesteuerte Schallwandlerveränderung zur Körperoberfläche des Kapillargebietes ist zusätzlich zum Tiefenfenster eine Ausblendung von Blutströmungsgeschwindigkeiten, wie sie aus grossen Gefäßen stammen, möglich. Die hieraus gewonnenen Signale werden nach einer Frequenzanalyse digital in Form eines Histogramms dargestellt und charakterisieren semiquantitativ den Transport der Eryhrozyten.

EP 0 016 399 A1

Croydon Printing Company Ltd.

P 29 11 258.1
AT 22.3.79

Rudolf Mauser
Haussmannstr. 146 B
7000 Stuttgart 1

Prof.Dr.-Ing. Horst Chmiel
Paracelsusstr. 14
7250 Leonberg

Vorrichtung zum noninvasiven Messen der Blutströmungsgeschwindigkeit nach der Ultraschall-Doppler-Effekt-Methode

Die Erfindung betrifft eine Vorrichtung zum noninvasiven Messen der
Blutströmungsgeschwindigkeit nach der Ultraschall-Doppler-Effekt -
Methode unter Verwendung von Ultraschall-Sendern/Empfängern für
den am strömenden Blut reflektierten Ultraschall und unter Verwendung von die Dopplerfrequenz-Verschiebung zwischen Sende- und
Empfangsfrequenz ermittelnden und auswertenden Einrichtung.

Derartige Vorrichtungen sind bereits bekannt (vgl. z.B. DOS 2461264).
Bei diesen Vorrichtungen bestehen u.a. folgende Nachteile: Die Geräte sind für die Messung der Blutströmungsgeschwindigkeit in relativ
großen Blutgefäßen vorgesehen. Sie arbeiten im Frequenzbereich von
5 bis 10 MHz, da für höhere Frequenzen die Dämpfung des Ultraschall-
signals im Körpergewebe zu groß würde, sodaß relativ tief liegende
Blutgefäße nicht mehr erfaßt würden. Durch die verwendeten Frequenzen lassen sich zwar die Strömungsgeschwindigkeiten in den großen Gefäßen (Größenordnung cm/s) erfassen, nicht jedoch Strömungsgeschwindigkeiten der Größenordnung mm/s wie sie im Bereich der kleinen Blut-

0016399

gefäße (Mikrozirkulation) vorliegen. Außerdem erlauben diese Vorrichtungen keine Differenzierung zwischen großen und kleinen Gefäßen. Zur Erfassung der Mikrozirkulation, insbesondere zur Detektion einer z.B. zeitlich abnehmenden oder zunehmenden Strömungsgeschwindigkeit in den Kapillargefäßen und Arteriolen eines ausgesuchten lokalen Gebiets bei praktisch unveränderter Strömung in den großen Gefäßen, stellt sich deshalb die folgende Aufgabe:

- Meßtechnische Erfassung kleiner Strömungsgeschwindigkeiten bei gleichzeitiger Unterdrückung der Signale von hohen Strömungsgeschwindigkeiten,

- weitgehende Unterscheidung der Signale von Kapillaren (einige Zehntel bis 1 mm/s) und Arteriolen (ungefähr 1 bis 20 mm/s)

- Beurteilung der zeitlichen Änderung des Korpuskelflusses (hier: Erythrozytenfluß), um so die Sauerstoffträger als Voraussetzung für den Sauerstoffaustausch in ihren zeitlichen Änderungen zu erfassen

- Nachweis von Erythrozytenaggregationen, die im Verlauf des Zusammenbruchs der Mikrozirkulation auftreten, mittels einer Laufzeitmessung (im gleichen Volumensbereich des Gewebes wie die Blutströmungsgeschwindigkeits-Messung).

Diese Aufgabe ist gemäß der Erfindung durch die im Patentanspruch 1 gekennzeichnete Vorrichtung gelöst. Dabei geht man von dem Gedanken aus, ein neuartiges elektronisches Tiefenfenster – mit dem die Signalanteile der Strömungsgeschwindigkeiten in den Endstrombahnen eines ausgewählten Volumenbereiches im oberflächennahen Körpergewebe (Tiefe bis zu etwa 3 mm ) von den Anteilen anderer Gebiete abgetrennt werden (wegen der geringen Tiefe kann die bei höheren Frequenzen stärkere Dämpfung nicht mehr in Kauf genommen sondern in positivem Sinn ausgenützt werden) – mit verschiedenen, an sich bekannten Merkmalen derart zu kombinieren, daß eine Erfassung der Blutströmungsgeschwindigkeiten in den kleinen und kleinsten Gefäßen in Unterscheidung zu diesen in größeren Gefäßen erstmals möglich ist;ferner, daß darüber hinaus der Einfluß des Hämatokrits auf die Blutströmungsgeschwindigkeit bestimmbar ist; außerdem, daß zusätzlich periodisch intermittierend (also im Effekt so gut wie gleichzeitig) das Auftreten von Erythrozytenaggregationen im gleichen Volumenbereich durch Laufzeitmessung mit Nadelimpulsen verfolgt wird.

Durch die Frequenzanalyse und die Darstellung in einem Vielkanalanalysator kann der Transport der Sauerstoffträger semiquantitativ charakterisiert werden. Damit wird es erstmals möglich, den Erfolg therapeutischer Maßnahmen (die z.B. pharmakologischer oder physikalischer Natur sein können) objektiv festzustellen. Ein wichtiges Anwendungsgebiet ist die Hämodilution, die heute in Intensivstationen ohne die Möglichkeit objektiver Kontrolle praktiziert werden muß. Das erfindungsgemäße Verfahren gestattet erstmals eine Optimierung der Hämodilution in der Antischocktherapie.

-4-

Die eigentliche Aufgabe des physiologischen Kreislaufs besteht darin, den Stoffaustausch zwischen Blut und Gewebe zu vollziehen, der im Bereich der kleinsten Gefäße, der sogenannten Endstrombahn oder Mikrozirkulation, stattfindet. Da die Mikrozirkulation als funktionelle und damit lebenswichtige Einheit angesehen werden muß, ist ihre Erfassung und Überwachung von großer medizinischer Wichtigkeit.

Die aus den kleinen Arterien hervorgehenden Arteriolen besitzen eine besonders reich an glatter Muskulatur durchzogene Wand und sind dadurch in der Lage, durch weitgehende Kontraktionen dieser Muskulatur den Strömungswiderstand zu variieren und sich sogar vollkommen zu verschließen.

Hinsichtlich der Anordnung der Gefäße untereinander gibt es derartig organspezifische Unterschiede, daß eine Verallgemeinerung nicht möglich ist, jedoch sind die nutritiven Kapillaren in großer Zahl angelegt und verlaufen hauptsächlich parallel zu den sogenannten Durchgangskanälen. Aus den Arteriolen entspringen die ebenfalls muskelhaltigen Metarteriolen und aus diesen die Kapillaren, die jedoch auch direkt aus den Arteriolen hervorgehen können.

Die Wand der Kapillaren besteht aus einer dünnen Schicht von Endothelzellen mit Basalmembran, die den Stoffaustausch zwischen dem Blut und der die Gewebezellen umgebenden interstitiellen Flüssigkeit ermöglicht. Aufgrund des kleinen Durchmessers in Verbindung mit der großen Anzahl und Dichte der Kapillargefäße ist zwar der Stoffaustausch begünstigt, stellt aber für das zirkulierende Blut gewisse Probleme dar.

Während das schnell fließende Blut optimal an diese Bedingungen angepaßt ist, besteht bei langsamer Strömung die Gefahr einer Verlegung
der engsten nutritiven Kapillaren durch Blutbestandteile oder Endothelschwellungen. Nach Durchströmung der Kapillaren fließt das Blut über
die Venolen, die sich ihrerseits zu größeren Venen sammeln, dem rechten Vorhof des Herzens zu. Während der mittlere Druck in den kleinen
Arterien noch zwischen 70 - 100 mm Hg beträgt, kommt es längs der
Arteriolen zu einem steilen Druckabfall, sodaß am Anfang der Kapillaren nurmehr ca. 30 mm Hg und an deren Ende nur noch 12 - 20 mm Hg
herrscht. Pulsatile Strömungen sowie ein derartiger Druck sind noch in
den Arteriolen und in den Anfangsstellen der Kapillaren nachzuweisen,
jedoch nicht mehr in deren weiterem Verlauf.

Da der Innendurchmesser der Blutkapillaren kleiner als jener der Blutkörperchen sein kann, erfolgt die Passage dieser meist nur in deformiertem Zustand. Die Deformierbarkeit der Erythrozyten hängt unter anderem
von der effektiven Viskosität sowie der noch nicht restlos geklärten Wechselwirkung zwischen Wand- und Blutkörperchenoberfläche ab, durch die
die Gleitfähigkeit verbessert wird. In peripheren Teilgebieten des Kreislaufs, z.B. einer Extremität, treten meist Nichtlinearitäten zwischen der
Beziehung Durchströmungsdruck und Stromstärke auf, die zum Teil auf
die rheologischen Eigenschaften des Blutes, hauptsächlich jedoch auf
das Verhalten der Widerstandsgefäße zurückzuführen sind. Bleibt der
Kontraktionszustand der glatten Muskulatur der Arteriolen konstant, so
erweitern sich bei einer Steigerung des Druchströmungsdrucks die Arteriolen und Kapillaren und der Strömungswiderstand nimmt ab. Gleich- zeitig vergrößert sich die Stromstärke überproportional mit steigendem Durchströmungsdruck.

Wird der Durchströmungsdruck im unteren Bereich der I-P-Beziehung gesenkt, so sistiert die Strömung bereits zu einem Zeitpunkt, wenn der Durchströmungsdruck noch größer als Null ist. Die Ursache hierfür liegt hauptsächlich darin, daß nunmehr die zur Konstriktion des Gefäßes führende aktive Spannung der glatten Muskulatur die das Gefäß dehnende Wirkung des transmuralen Druckes überwiegt. Wird ein bestimmter kritischer Verschlußdruck unterschritten, so kollabiert das Gefäß. Die einzelnen Teilgebiete und Organe passen großteils selbsttätig ihre Durchblutung den wechselnden Bedürfnissen an, indem lokale Stoffwechselprodukte, Metaboliten, den Kontraktionszustand der glatten Muskulatur der Arteriolen herabsetzen und damit auch den lokalen Strömungswiderstand. Die dadurch zustandekommende Mehrdurchblutung sorgt für eine Konzentrationsverminderung der Metaboliten, wodurch die Durchblutung wieder sinkt. Diese lokalen Vorgänge tragen die typischen Kennzeichen einer Regelstrecke, deren geschlossener Wirkungskreis mit einer Gegenkopplung versehen ist.

Bei akuten hämodynamischen Störungen, wie sie z.B. aufgrund eines Schocks auftreten, wird die Kapillardurchblutung in einem Ausmaß herabgesetzt, daß sich eine Gewebshypoxie (Sauerstoffmangel) entwikkelt, die zu funktionellen und morphologischen Veränderungen führt.

Bei jedem schweren Schock wird innerhalb kürzester Zeit thromboplastisches (gerinnungsförderndes) Material freigesetzt, das in Sekunden zu einer Verklebung von Thrombozyten (Blutplättchen) und als Folge davon zu einer Erythrozytenaggregation (Anhäufung roter Blutkörperchen) führt.

Durch die immer größer werdende Erythrozytenaggregation kommt es infolge einer Albumin-Globulin-Relation zu Gunsten der großmolekularen Globuline zu einer Viskositätserhöhung (reduzierte Elastizität und blood-sludge (Blutschlamm).

Diese verschlechterte Fließbarkeit des Blutes führt zu einer Erythrostase (Stauung) mit Verstopfung der Venolen (kleine venöse Gefäße) und Kapillaren (kleinste Blutgefäße). Infolge Blutdruckabfalls und Arteriolenkonstriktion (Verengung) ist die Strömungsgeschwindigkeit im Kapillargebiet derart stark reduziert, daß es zum Erliegen der Mikrozirkulation kommt. Die dabei eintretende Hypoxydose (verminderte Zellatmung infolge reduzierten Sauerstoffdrucks) führt über Gewebsazidose (Säureanstieg) und lokale Stoffwechselschädigung zu schwerer Beeinträchtigung des Gesamtorganismus.

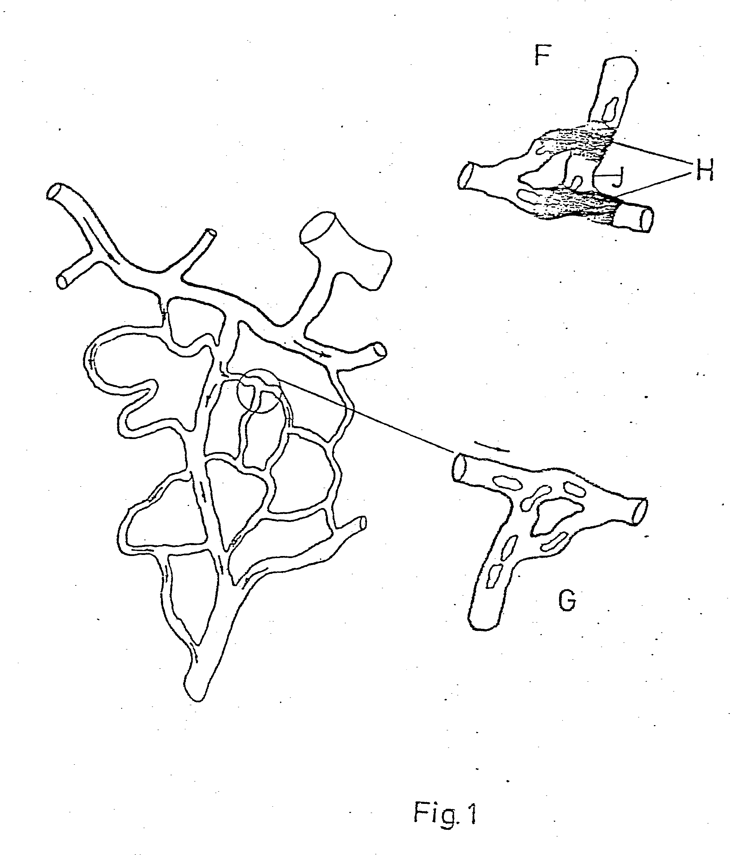

Fig. 1 zeigt schematisch einen Teil aus einem Kapillarbett mit zwei Ausschnittsvergrößerungen F und G. Während die Vergrößerung G eine normale Erythrozytenbewegung zeigt, ist in F ein Schockzustand dargestellt, der sich in Form von einer Erythrozytenaggregation H und einem Plasmaeinschluß J äußert. Der Durchmesser der dargestellten Gefäße bewegt sich zwischen 6 und 40 µ (Kapillare und Arteriolen) bei einer Wandstärke zwischen 1 und 20 µ.

Um eine maximale Shiftfrequenz (Dopplerfrequenz $\Delta$ f) der in den Endstrombahnen ca. 1 mm/s betragenden Blutströmungsgeschwindigkeit meßtechnisch zu erfassen, sind hierfür Ultraschallfrequenzen von mindestens
20 MHz für die Blutströmungsgeschwindigkeit und Nadelimpulse z.B. mit
einer Breite von etwa 20 ns zum Erfassen einer Aggregation erforderlich.

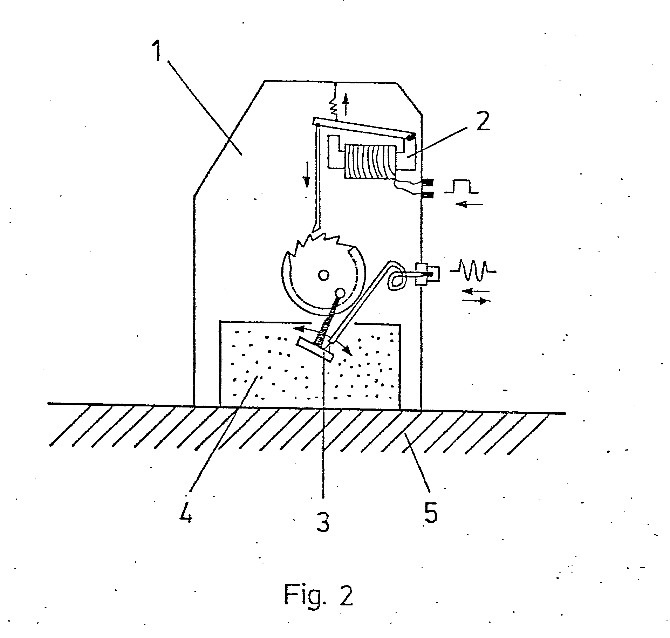

Hierzu wird ein ca. 1 mm$^2$ großer Piezokristall aus Blei-Zirkonat-Titanat
oder einem anderen gleichwertigen kristallinen oder amorphen Material
unter Zwischenschaltung einer mit akustischem Koppelgel gefüllten Vorlaufstrecke über der zu untersuchenden Stelle appliziert. Der Sender/
Empfängerkristall ist gemäß Fig.2 in einer Haltevorrichtung 1 angeordnet, in der ein Mikroschritt-Motor derart angebracht ist, daß eine Schallwinkel-Korrektur von 15° bis 30° in 1° Schritten zur Körperoberfläche
durchgeführt werden kann.

Durch diese Einrichtung wird erreicht, daß bei ungünstiger Lage des
Sender/Empfängerkristalls zum Gefäßbett und der damit verbundenen
Detektion von Strömungsgeschwindigkeitsanteilen aus größeren Gefäßen
eine den tatsächlichen Geschwindigkeitsverhältnissen entsprechende Verfälschung weitestgehend ausgeschaltet wird. Im Normalfall erfolgt die
Einschallung mit einem Winkel von 15° zur Körperoberfläche, dies kann
jedoch dazu führen, daß ein größeres Gefäß seitlich derart getroffen und
eine Strömungsgeschwindigkeit detektiert wird, die nicht jener der tatsächlichen Vorgänge in den Kapillaren entspricht. Durch die Veränderung der Winkelstellung des Sender/Empfängerkristalls über den Schrittmotor und/oder durch Verschiebung der Haltevorrichtung 1 wird dieser
Störfaktor eliminiert.

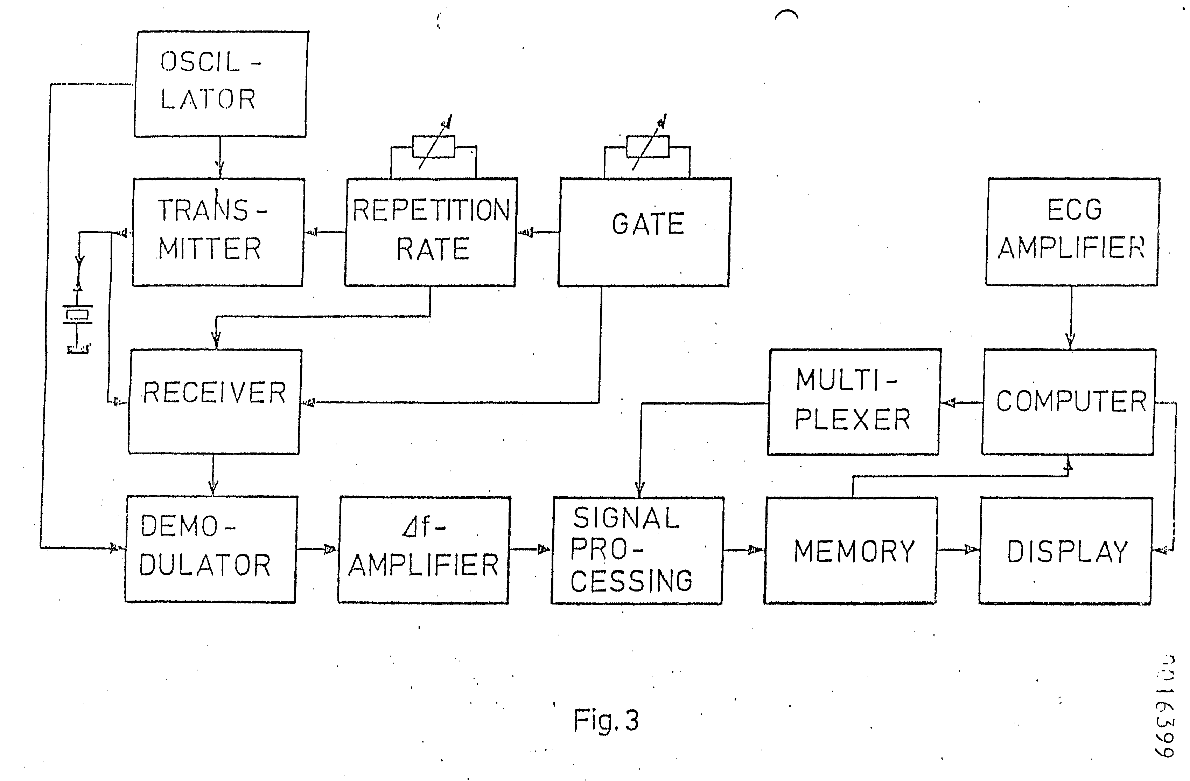

Fig.3 zeigt ein Blockschaltbild der Sende- und Empfangselektronik für das Messen der Strömungsgeschwindigkeit. Für die Steuerung des Schrittmotors wird in einer Vergleichsstufe der Elektronik (receiver) eine Frequenz vorgegeben und mit der empfangenen $\Delta$ f Frequenz verglichen; ist dieser Wert höher, erfolgt eine Schrittmotorsteuerung so lange, bis die vorgegebene Frequenz unterschritten wird. Da in den von medizinischer Sicht relevanten Gefäßregionen prinzipiell nur eine Strömungsrichtung, nämlich von der arteriellen zur venösen Seite stattfinden kann, jedoch durch quer- und gegenläufig liegende Gefäße auch ein vorgetäuschter "Rückfluß" in Form einer negativen $\Delta$ f registriert wird, erfolgt bei der Demodulation die Weiterverarbeitung nur bei den Frequenzen mit positivem Vorzeichen, entsprechend jener Blutgefäße, deren Strömung sich auf den Sender/Empfängerkristall zu bewegt.

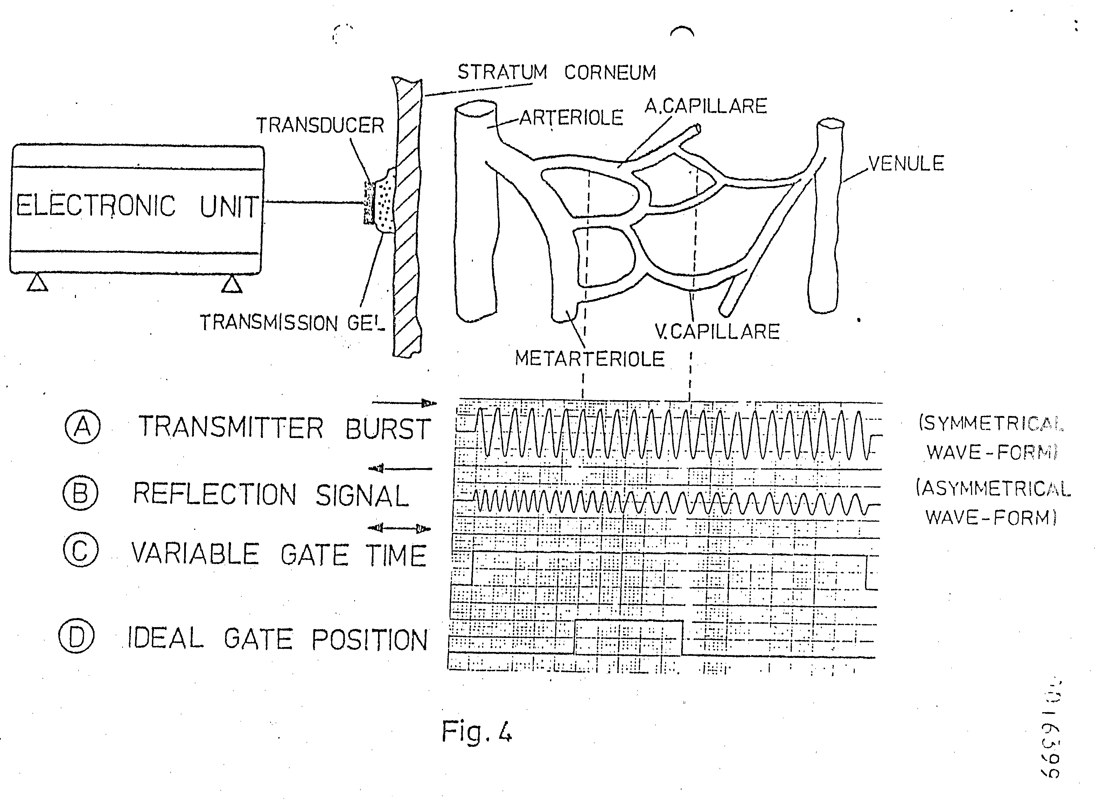

In Fig.4 ist A das gesendete Impulspaket und B das Echosignal entsprechend dem darüber gezeichneten Gefäßbett. C zeigt die Variationsbreite des auf D dargestellten elektronischen gate. Die ideale Position ist der Gefäßabschnitt zwischen den gestrichelten Linien, da hier keine größeren Gefäße vorhanden sind. Die Breite des gate ist unabhängig von seiner Position in Stufen von 0,001 mm digital einstellbar und kann auf die Länge des Sendeimpulspaketes abzüglich der ersten Wellenzüge bzw. Nulldurchgänge (entsprechend den Einschwingzeiten des Sender/Empfängerkristalls und des Verstärkers) des Echosignals ausgedehnt werden.

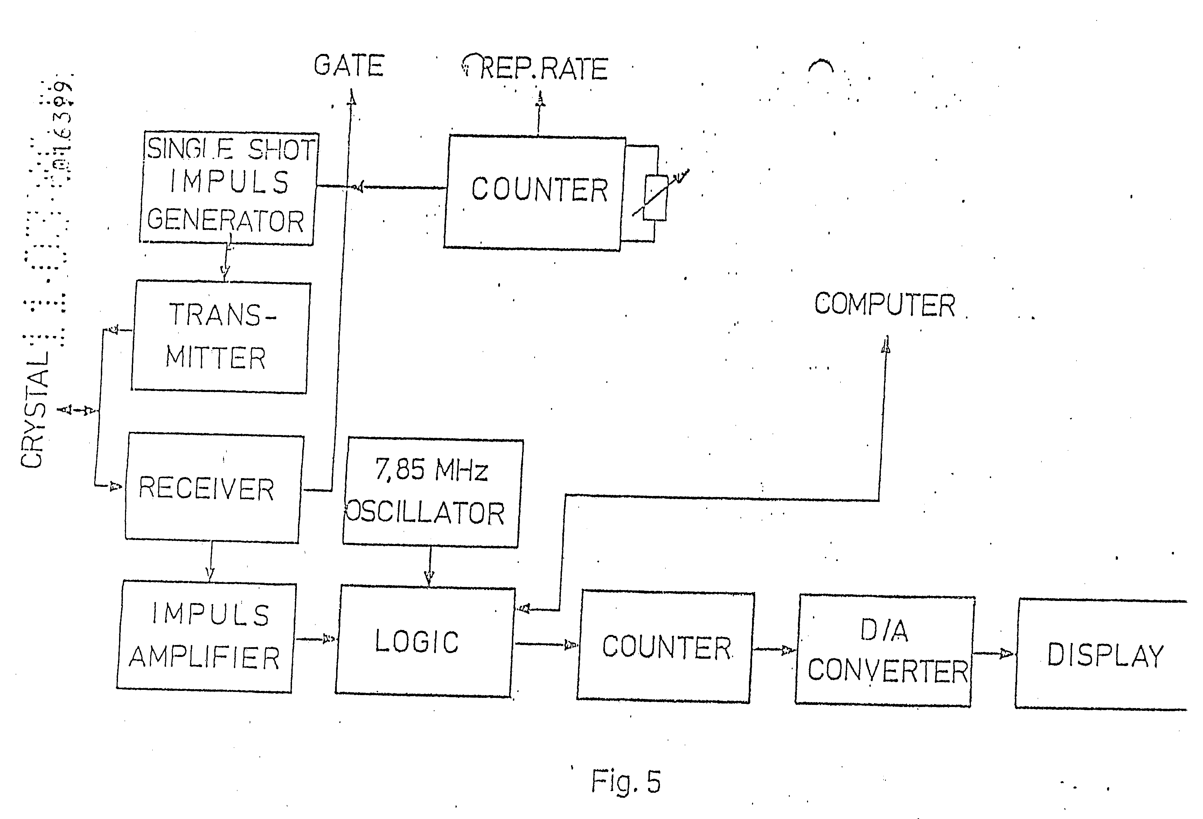

Fig.5 zeigt den stark vereinfachten Stromlaufplan der Meßeinrichtung für die Messung der Blutströmungsgeschwindigkeit. Ein Oszillator erzeugt eine der Resonanzfrequenz des Sender/Empfängerkristalls entsprechende Hochfrequenz, die über den gepulst betriebenen Sender (transmitter) auf den Sender/Empfängerkristall geschaltet wird. Das Puls-Pausenverhältnis wird über die repetition-rate-Einheit im Verhältnis 1:1 bis 1:16 kontinuierlich und reproduzierbar eingestellt und erwirkt die Umschaltung des Sender/Empfängerkristalls von Senden auf Empfang. Das vom Empfänger in der gleichen Dauer des gesendeten Signals aufgenommene Echo wird auf die eingestellte gate-Breite – entsprechend der Gewebetiefe – reduziert und dem vom Oszillator synchronisierten Demodulator zugeführt. Nach Abtrennung der Hochfrequenz und der einem vorgetäuschten "Rückfluß" zugeordneten negativen $\Delta$ f erfolgt eine Signalverstärkung und Tiefpassfilterung ( $\Delta$ f amplifier).

Die der Strömungsgeschwindigkeit proportionale $\Delta$ f von 0,1 bis 1000 Hz wird in der nachfolgenden Prozeßeinheit (signal processing) zwischengespeichert und in schmalbandige Frequenzkanäle unterteilt, die von einem rechnergesteuerten Multiplexer abgefragt, aufsummiert und im memory abgespeichert werden. Vom gleichzeitig getrennt gemessenen EKG erfolgt unter Berücksichtigung der ebenfalls registrierten Respiration eine rechnergesteuerte Abfrage des Speichers (memory), dessen Inhalt, soweit er der zeitlichen Zuordnung der Pulswelle entspricht, in Form eines Frequenzhistogramms (display) zur Darstellung gebracht wird. Der Ablauf dieser Steuerung erfolgt dahingehend, daß durch die R-Zacke (Systole) im EKG ein Triggerimpuls ausgelöst wird, der seinerseits eine Verzögerungsschaltung (computer) mit einer Q-T-Zeit im EKG entsprechenden metastabilen Zeit triggert. Die Rückflanke dieser Einheit öffnet
eine der hämodynamischen Pulswellenbreite entsprechende Torschaltung
(gate). Da nur in diesem Zeitraum eine relevante Blutstrombewegung in
der Mikrozirkulation stattfindet, erfolgt die Auswertung der geschwindigkeitsproportionalen $\Delta$ f nur in diesem Zeitabschnitt. Dies hat unter anderem den Vorteil, daß durch die extrem hohe Empfindlichkeit der Meßeinrichtung alle Störsignale zwischen zwei Systolen ausgeblendet werden und
nur für die in der Diastole aufscheinenden und medizinisch interessanten
Vorgänge ausgewertet werden.

Durch diese Einrichtung ist desweiteren die Möglichkeit gegeben, daß
eine vorzeitige Kontraktion (Extrasystole), soweit diese sich in der Peripherie manifestiert, im Geschwindigkeitshistogramm erkennbar ist und sich deutlich von Veränderungen, wie sie respiratorisch bedingt
auftreten, unterscheidet.

Nach jedem n-ten Sendeimpulspaket (z.B. n = 5000) zur Messung der Blutströmungsgeschwindigkeit erfolgt über denselben Sender/Empfängerkristall
eine Impulslaufzeitmessung zur Detektion von Erythrozytenaggregationen.
Hierzu wird eine in Fig.5 dargestellte Schaltungsanordnung verwendet.
Ein von der repetition-rate-Einheit in Fig.3 getriggerter Vorwahlzähler
(counter) steuert über den single shot Impulsgenerator den Sender (transmitter), der einzelne Nadelimpulse (z.B. einen Sinushalbwellenimpuls)
entsprechend der Resonanzfrequenz des transducers (crystal) auf den Sender/Empfängerkristall (crystal) schaltet. Bei Auftreten einer Erythrozyten- aggregation im eingestellten gate-Bereich kommt es zu einem Echo, das über den Empfänger (receiver) dem Impulsverstärker (impule amplifier) zugeführt wird. Entsprechend einer Schallgeschwindigkeit von 1570 m/s der Ultraschallwellen in Blut und Gewebe wird für die Laufzeitmessung ein Quarzoszillator (hier 7,85 MHz Oszillator) mit einer ausgewählten festen Frequenz (z.B. 7,85 MHz entsprechend der halben Wegstrecke) als Zählimpulssignal verwendet.

Mit Abgabe des Sendeimpulses wird über die Logic das z.B. 7,85 MHz Signal auf den Zähler geschaltet und bei Eintreffen eines Echos gestoppt. Das angezeigte digitale Meßergebnis entspricht direkt der Tiefe der Erythrozytenaggregation. Nach der D/A-Convertierung erfolgt eine analoge Darstellung (display), die sich je nach Bewegung der Aggregation ausdrückt.

Da es bei der Detektion einer Aggregation zu einer drastischen Änderung der Blutströmungsgeschwindigkeit kommt (Abfall und/oder Ansteigen), kann auf beiden Kanälen (Fig.4 und Fig.5) der Anzeige der Verlauf des Vorganges zueinander bezogen werden und damit ein rechtzeitiges Vorbeugen durch den Arzt erfolgen und unter Umständen ein Schockeintritt verhindert werden. Erfolgt keine Detektion mittels der Laufzeiteinrichtung (Fig.5), löscht sich der Zähler nach der vorgewählten Zeit selbständig und bleibt bis zum näch- sten Sendeimpuls empfangsbereit.

Rudolf Mauser
Hausmannstr. 146 B
7000 Stuttgart 1

Prof.Dr.-Ing. Horst Chmiel
Paracelsusstr. 14
7250 Leonberg

## PATENTANSPRÜCHE

1. Vorrichtung zum noninvasiven Messen der Blutströmungsgeschwindigkeit nach der Ultraschall-Doppler-Effekt-Methode
unter Verwendung von Ultraschall-Sendern/Empfängern für
den am strömenden Blut reflektierten Ultraschall und unter
Verwendung von die Dopplerfrequenz-Verschiebung zwischen Sende- und Empfangsfrequenz ermittelnden und auswertenden Einrichtung,

dadurch gekennzeichnet,

daß zum Messen der Blutströmungsgeschwindigkeit im Bereich
der kleinen und kleinsten Gefäße (Mikrozirkulation) und/oder
zum Erfassen einer Erythrozyten-Aggregation eine Vorrichtung
vorgesehen ist, die aufweist

a) einen (einzigen) Sender-Empfänger-Kristall, (3) dessen
Winkelstellung zur Hautfläche etwa zwischen 15° und
30° wahlweise einstellbar ist,

b) Schalteinrichtungen zur Aussendung von vorzugsweise periodisch aufeinanderfolgenden Impulspaketen mit einer Ultraschallfrequenz von mindestens 20 MHz für die Blutströmungsgeschwindigkeit der Mikrozirkulation und von Nadelimpulsen z.B. mit einer Breite von etwa 20 ns zum Erfassen einer Erythrozyten-Aggregation,

c) Schalteinrichtungen zum Empfang und zur Verstärkung der reflektierten Signale, durch die bei der Demodulation alle negativen Dopplerfrequenzanteile ($\Delta f$ gleich Sendefrequenz minus Empfangsfrequenz) ausgeblendet werden,

d) eine Vergleichseinrichtung, die die empfangene Dopplerfrequenz ($\Delta f$) mit einer vorgegebenen Frequenz vergleicht, die der maximalen Strömungsgeschwindigkeit in der Mikrozirkulation entspricht,

e) eine Steuereinrichtung (gate), die als Tiefenfenster wirkt und die in einer vorbestimmbaren Gewebetiefe ein vorbestimmbares Meßvolumen unabhängig von der Wiederholfrequenz erfaßt.

2. Vorrichtung nach Patentanspruch 1.

dadurch gekennzeichnet,

daß die Winkelstellung des Sender-Empfängerkristalls automatisch, z.B. durch einen Schrittmotor (2) einstellbar ist.

3. Vorrichtung nach Patentansprüchen 1. und 2.

dadurch gekennzeichnet,

daß der Steuereinrichtung (gate) eine Zähleinrichtung zugeordnet ist, die von dem empfangenen Echo die ersten Nulldurchgänge (entsprechend der Einschwingzeiten des Sender/Empfängerkristalls (3) und des Verstärkers) elektronisch ausblendet und dadurch eine infolge des Einschwingvorganges vorgetäuschte Dopplerfrequenz ( $\Delta$ f) eliminiert wird.

4. Vorrichtung nach Patentansprüchen 1. und 3.

dadurch gekennzeichnet,

daß den Schalteinrichtungen zur Aussendung von Impulspaketen eine Steuereinrichtung (repetition rate/gate) zugeordnet ist, mittels derer die Länge des Sendeimpulspaketes eingestellt und dadurch die abgestrahlte Leistung und die Eindringtiefe veränderbar ist.

5. Vorrichtung nach Patentansprüchen 1. und 4.

dadurch gekennzeichnet,

daß die Haltevorrichtung (1) für den Sender-Empfängerkristall (3) so ausgebildet ist, daß eine akustische Vorlaufstrecke zwischen Sender/

-4-

Empfängerkristall und der Oberfläche von Körpergeweben
(z.B. Serosa, Mucosa oder freigelegtes Organ, z.B. Niere)
einstellbar ist.

6. Vorrichtung nach den Patentansprüchen 1. und 5.

dadurch gekennzeichnet,

daß die Wiederholfrequenz für die auszusendenden Impulspakete unabhängig von der Breite und Lage des Tiefenfensters
einstellbar ist.

7. Vorrichtung nach Patentansprüchen 1. und 6.

dadurch gekennzeichnet,

daß den Schalteinrichtungen zum Empfang der reflektierten
Signale eine Steuereinrichtung zugeordnet ist ( receiver),
die abhängig von dem getrennt aufgenommenen EKG-Signal
der Systole ein vorgegebenes Auswertintervall zeitverzögert
auslöst.

8. Vorrichtung nach Patentansprüchen 1. und 7.

dadurch gekennzeichnet,

daß eine den Schalteinrichtungen zum Empfang nachgeordnete
Schalteinrichtung vorgesehen ist, die das empfangene Dopplerfrequenzsignal entsprechend der physiologischen Strömungsrichtung in einer Frequenzanalyse zerlegt und die Teilfrequenzen so aufsummiert, daß ein Frequenzspektrum entsprechend dem tatsächlichen Geschwindigkeitsbild in der
Mikrozirkulation dargestellt wird.

9. Vorrichtung nach Patentansprüchen 1. und 8.

dadurch gekennzeichnet,

daß den Schalteinrichtungen zur Aussendung von Impulspaketen eine Steuereinrichtung (counter) zugeordnet ist,
die nach jedem n-ten Impulspaket (z.B. n = 5000) einen Nadelimpuls aussendet, wobei n unabhängig von den Einrichtungen zur Geschwindigkeitsmessung einstellbar ist.

10. Vorrichtung nach Patentansprüchen 1. und 9.

dadurch gekennzeichnet,

daß eine elektronische Auswerteeinrichtung das Frequenzspektrum so aufarbeitet, daß eine Beurteilung der zeitlichen Veränderung des Erythrozyten-Flusses, z.B. bei der Hämodilution zu
deren Optimierung, möglich ist.

Fig. 1

Fig. 2

Fig. 3

STRATUM CORNEUM

TRANSDUCER

A.CAPILLARE

ARTERIOLE

VENULE

ELECTRONIC UNIT

TRANSMISSION GEL

V.CAPILLARE

METARTERIOLE

(A) TRANSMITTER BURST

(SYMMETRICAL WAVE-FORM)

(B) REFLECTION SIGNAL

(ASYMMETRICAL WAVE-FORM)

(C) VARIABLE GATE TIME

(D) IDEAL GATE POSITION

Fig. 4

Fig. 5

# EUROPÄISCHER RECHERCHENBERICHT

**Europäisches Patentamt**

Nummer der Anmeldung

EP 80 10 1224

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 4 109 642 (J.M. REID & M.P. SPENCER/ INSTITUTE OF APPLIED PHYSIOLOGY AND MEDICINE) <br><br> * Zusammenfassung; Spalte 1, Zeile 52 bis Spalte 2, Zeile 52; Spalte 3, Zeilen 25-65; Spalte 4, Zeilen 37-60; Spalte 6, Zeilen 12-60; Spalte 8, Zeile 48 bis Spalte 9, Zeile 58; Spalte 11, Zeilen 17-48; Abbildungen 1-14 * <br><br> -- | 1,5 |
| | FR - A - 2 159 076 (AMERICAN OPTICAL CORP.) <br><br> * Seite 2, Zeilen 1-39; Seite 4, Zeile 3 bis Seite 6, Zeile 13; Seite 9, Zeile 34 bis Seite 10, Zeile 28; Seite 11, Zeilen 6-33; Abbildungen 1-11 * <br><br> -- | 1,5,8 |
| | FR - A - 2 309 865 (THE COMMON-WEALTH OF AUSTRALIA) <br><br> * Seite 9, Zeile 24 bis Seite 11, Zeile 20; Abbildungen 1-4 * <br><br> -- | 1,2,5 |
| | US - A - 3 778 756 (J.M. HOUSTON & J.D. KINGSLEY/ GENERAL ELECTRIC COMP.) <br><br> * Zusammenfassung; Spalte 2, Zeilen 17-35; Spalte 7, Zeilen 22-58; Spalte 9, Zeilen 22-62; Spalte 12, Zeilen 3-40; Abbildungen 3-5 * <br><br> -- <br><br> ./. | 1,4,5 |

**KLASSIFIKATION DER ANMELDUNG (Int.Cl.³)**

A 61 B 10/00

**RECHERCHIERTE SACHGEBIETE (Int. Cl.³)**

A 61 B 10/00
G 01 S 9/66

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 26-06-1980 | RIEB |

EPA form 1503.1   06.78

0016399

Europäisches
Patentamt

**EUROPÄISCHER RECHERCHENBERICHT**

Nummer der Anmeldung

Er 65 10 12..

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | US - A - 3 778 757 (J.M. HOUSTON/ GENERAL ELECTRIC COMP.) <br><br> * Zusammenfassung; Spalte 2, Zeilen 28-48; Spalte 11, Zeile 19 bis Spalte 12, Zeile 60; Spalte 13, Zeile 34 bis Spalte 14, Zeile 17; Spalte 16, Zeile 58 bis Spalte 17, Zeile 35; Abbildungen 5-8 * <br><br> -- <br><br> DE - A - 2 151 347 (SIEMENS AG) <br><br> * Seite 3, Zeile 7 bis Seite 6, Zeile 8; Abbildung 1 * <br><br> -- <br><br> DE - A - 1 907 320 (SIEMENS AG) <br><br> * Seite 2, Zeile 9 bis Seite 4, Zeile 10; Seite 5, Zeilen 1-11; Abbildungen * <br><br> -- <br><br> US - A - 3 605 724 (J.J. FLAHERTY/ MAGNAFLUX CORP.) <br><br> * Spalte 1, Zeilen 12-32; Spalte 1, Zeile 55 bis Spalte 3, Zeile 19; Spalte 3, Zeile 44 bis Spalte 4, Zeile 31; Spalte 5, Zeilen 24-63; Spalte 7, Zeilen 41-59; Abbildungen 1-4 * <br><br> -- <br><br> US - A - 4 106 492 (W.H. SCHUETTE et al./ USA SECRETARY OF THE DE- PARTMENT OF HEALTH, EDUCATION AND WELFARE) <br><br> * Zusammenfassung; Spalte 3, Zeilen 19-64; Spalte 5, Zeilen <br> ./. | 1,4,5 <br><br><br><br><br><br><br><br><br><br><br> 1,7 <br><br><br><br><br><br> 1,8 <br><br><br><br><br><br><br> 1,7 | RECHERCHIERTE SACHGEBIETE (Int. Cl. 3) |

EPA Form 1503.2   06.78

Europäisches
Patentamt

**EUROPÄISCHER RECHERCHENBERICHT**

Nummer der Anmeldung

EP 80 10 12..
-3-

| | EINSCHLÄGIGE DOKUMENTE | | KLASSIFIKATION DER ANMELDUNG (Int.Cl. ³) |
|---|---|---|---|
| **Kategorie** | **Kennzeichnung des Dokuments mit Angabe, soweit erforderlich, der maßgeblichen Teile** | **betrifft Anspruch** | |
| | 55-68; Abbildung 1 *  --  US - A - 4 031 743 (G. KOSSOFF et al./ THE COMMONWEALTH OF AUSTRA-LIA)  * Zusammenfassung; Spalte 1, Zeilen 8-27; Spalte 2, Zeilen 61-66; Spalte 3, Zeilen 44-58; Spalte 4, Zeile 61 bis Spalte 5, Zeile 25; Abbildungen 4,5 *  -- | 1,5 | |
| P | DE - A - 2 758 039 (H. CHMIEL & R. MAUSER)  * Ansprüche 1,4-6; Seite 4, Zeilen 8-32; Seite 5, Zeile 29 bis Seite 6, Zeile 37; Abbildungen 1,2 *  -- | 1,3 | **RECHERCHIERTE SACHGEBIETE (Int. Cl. ³)** |
| PE | EP - A - 0 008 517 (GENERAL ELECTRIC COMP.)  * Zusammenfassung; Seite 4, Zeile 5 bis Seite 5, Zeile 20; Seite 6, Zeile 16 bis Seite 8, Zeile 18; Seite 14, Zeile 15 bis Seite 17, Zeile 11; Ab-bildungen 1-7 *  ---- | 1,8,10 | |